# EUROPEAN PATENT SPECIFICATION

(11) **EP 3 977 130 B1**
(45) Date of publication and mention of the grant of the patent: **24.07.2024**
(21) Application number: 20728790.5
(22) Date of filing: 03.06.2020
(51) Int. Cl.: G01N 33/574

(54) **METHODS FOR MODULATING A TREATMENT REGIMEN**
VERFAHREN ZUR MODULATION EINES BEHANDLUNGSSCHEMAS
PROCÉDÉS DE MODULATION D'UN RÉGIME DE TRAITEMENT

(30) Priority: 03.06.2019 EP 19177991; 03.06.2019 EP 19305711
(43) Date of publication of application: 06.04.2022
(73) Proprietor: Institut National de la Santé et de la Recherche Médicale (INSERM), 75013 Paris (FR); Assistance Publique Hôpitaux de Paris, 75004 Paris (FR); Université Paris Cité, 75006 Paris (FR); Association Gercor, 75011 Paris (FR); Veracyte, 13009 Marseille (FR)
(72) Inventor: HERMITTE, Fabienne, 13600 Ceyreste (FR); VERNEREY, Dewi, 25115 Pouilley les Vignes (FR); HENRIQUES, Julie, 25410 Ferrières les bois (FR); ANDRE, Thierry, 75015 Paris (FR); TAIEB, Julien, 75013 Paris (FR); PAGES, Franck, 75006 Paris (FR); GALON, Jérôme, 75006 Paris (FR); CATTEAU, Aurélie, 13009 Marseille (FR)
(74) Representative: Cabinet Becker et Associés
(86) International application number: PCT/EP2020/065289
(87) International publication number: WO 2020/245155

(56) References cited:
- WO-A1-2017/194556
- WO-A1-2018/027223
- WO-A2-2012/038068
- CN-A- 106 501 516
- ANONYMOUS: "Immunoscore: a test to improve the care and treatment of colon cancer", INSERM: PRESS INFORMATION, 30 May 2018 (2018-05-30), XP055713975, Retrieved from the Internet <URL:https://presse.inserm.fr/en/immunoscore-a-test-to-improve-the-care-and-treatment-of-colon-cancer/31545/> [retrieved on 20200713]
- BERNHARD MLECNIK ET AL: "Immunoscore as a predictor of response to chemotherapy in stage II and stage III colon cancer", JOURNAL FOR IMMUNOTHERAPY OF CANCER, BIOMED CENTRAL LTD, LONDON, UK, vol. 3, no. 2, 4 November 2015 (2015-11-04), pages 1, XP021235679, DOI: 10.1186/2051-1426-3-S2-P89
- JEROME GALON: "Immunoscore clinical utility to identify good prognostic colon cancer stage II patients with high-risk clinico-pathological features for whom adjuvant treatment may be avoided.", JOURNAL OF CLINICAL ONCOLOGY, vol. 37, no. 4, 29 January 2019 (2019-01-29), pages 487, XP055714040
- ANONYMOUS: "Immunoscore Might Help Spare Patients With High-Risk Stage II Colon Cancer From Unnecessary Chemotherapy", 23 January 2019 (2019-01-23), XP055714046, Retrieved from the Internet <URL:https://dailynews.ascopubs.org/do/10.1200/ADN.19.190076/full/> [retrieved on 20200713]
- J. GALON ET AL: "Immunoscore and Immunoprofiling in cancer: an update from the melanoma and immunotherapy bridge 2015", JOURNAL OF TRANSLATIONAL MEDICINE, vol. 14, no. 1, 20 September 2016 (2016-09-20), XP055656087, DOI: 10.1186/s12967-016-1029-z
- F. PAGÈS ET AL: "Prognostic and predictive value of the Immunoscore in stage III colon cancer patients treated with oxaliplatin in the prospective IDEA France PRODIGE-GERCOR cohort study", ANNALS OF ONCOLOGY., vol. 31, no. 7, 1 July 2020 (2020-07-01), NL, pages 921 - 929, XP055713943, ISSN: 0923-7534, DOI: 10.1016/j.annonc.2020.03.310
- TAKEHIRO OTOSHI ET AL: "Possible Biomarkers for Cancer Immunotherapy", CANCERS, vol. 11, no. 7, 3 July 2019 (2019-07-03), pages 935, XP055713959, DOI: 10.3390/cancers11070935

## Description

The present invention relates to methods for determining, modulating or adjusting a treatment regimen with a chemotherapeutic agent in a patient affected with a cancer.

### Background of the invention

As explained in detail by Galon et al (Cancer classification using the Immunoscore: a worldwide task force J Transl Med. 2012; 10: 205), prediction of clinical outcome in cancer is usually achieved by histopathological evaluation of tissue samples obtained during surgical resection of the primary tumor. Traditional tumor staging (AJCC/UICC-TNM classification) summarizes data on tumor burden (T), presence of cancer cells in draining and regional lymph nodes (N) and evidence for metastases (M). Galon et al. have shown that the immune active or immune silent tumors associated with cytotoxic and memory T-cells, Th1 cells, and interferon-gamma (IFN-γ) signature are correlated with long-term survival or rapid recurrence respectively (Galon et al, Science, 2006;313(5795):1960-4; Camus et al,. Cancer Res. 2009;69(6):2685-93). The consensus Immunoscore^{®} categorizing inflamed and non-inflamed tumors was recently validated globally with profound clinical implications (Pages et al, Lancet. 2018;391(10135):2128-39). Mlecnik et al. (J Immunother Cancer. 2015; 3(Suppl 2): P89) discloses Immunoscore as a predictor of response to chemotherapy in stage II and stage III colon cancer.

While methods for determining sensitivity of a patient toward an anti-tumoral therapy have been proposed, there is still a need for tools which would aid the physician in modulating the therapy in a most efficient manner.

### Summary of the invention

It is herein provided a method for determining, modulating or adjusting a treatment regimen with a chemotherapeutic agent in a patient affected with a cancer, wherein said agent is able to leverage or promote a tumor-targeted immune response, preferably by causing immunological cell death (ICD) which method is as defined in the claims.

According to the invention, the clinical benefit of a prolonged period of chemotherapy, e.g. 6 months of chemotherapy, becomes more important the higher the IS gets.

The methods of the invention are performed *in vitro,* preferably before the patient is administered with the chemotherapeutic agent.

### Legends to the Figures

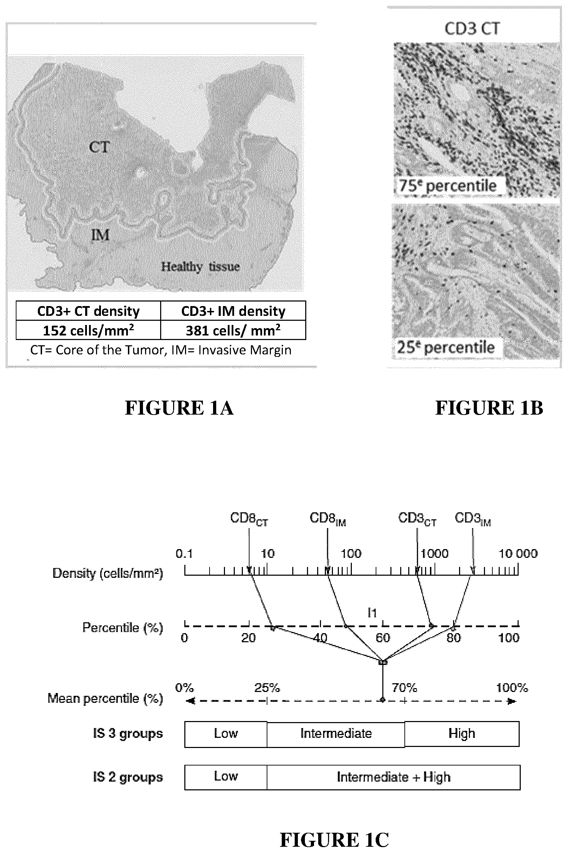

**Figure 1****. Immunoscore^{®} calculation method.** (A) Densities of CD3 and CD8 are quantified in the center (CT) and invasive margin (IM) of the tumor (CD3ct, CD8ct, CD3im, CD8im) using dedicated Immunoscore Analyzer software. Densities are reported on the pre-defined Percentile scale and mean Percentiles of the 4 markers is calculated to define the Percentile Immunoscore (from 0 to 100). Example of CT and IM region determined by the software are shown on the left. Example of IHC staining (CD3) and positive cell detection are shown on the right. On the example, the patient has an Intermediate Immunoscore (60%).

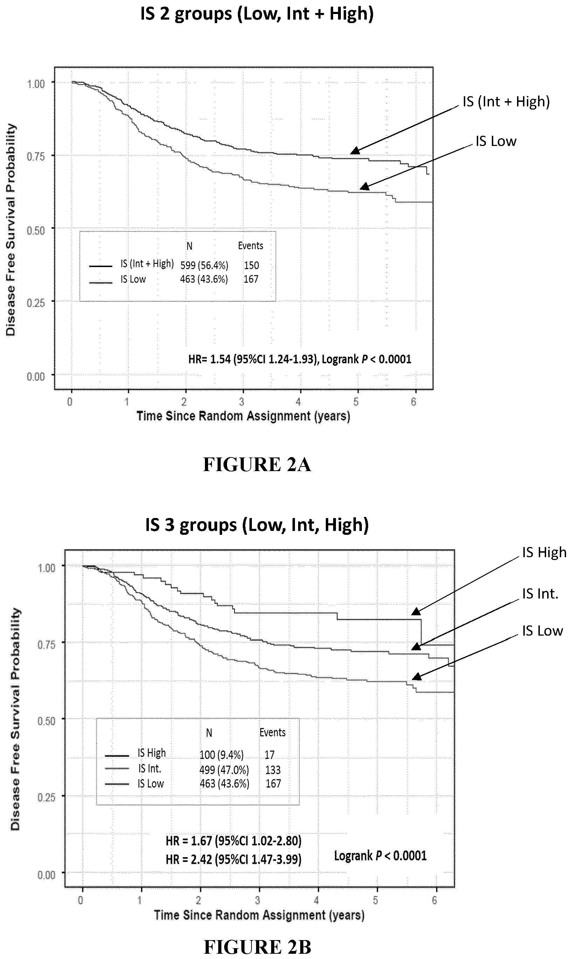

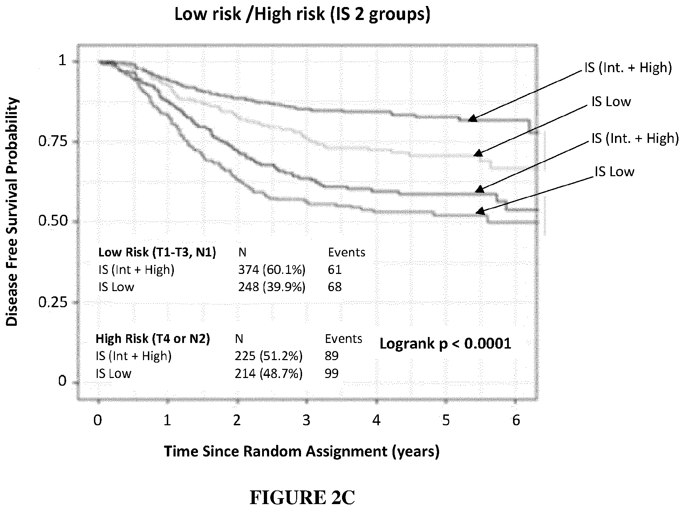

**Figure 2****. Prognostic value of the Immunoscore^{®} in Stage III cancer patients** (Kaplan Meier estimates of DFS)(A) Among patients' groups stratified by IS into two categories (Low versus Int+High), the 3-year DFS rates were 66.80% [95%CI 62.23-70.95] and 77.14% [95%CI 73.50-80.35] for IS Low and IS Int+High, respectively (p = 0.0001) (B) with IS in 3- categories, a 3-year DFS rate of 85% was observed in patients with IS High, vs 67% for patients with IS Low (p = 0.0001) NB: IS as continuous variable was also significantly associated with 3-year DFS (p<0.001). (C) The deleterious effect of IS Low in terms of DFS was higher in patients with T1-3 than in patients with T4 tumors (p=0.0212).

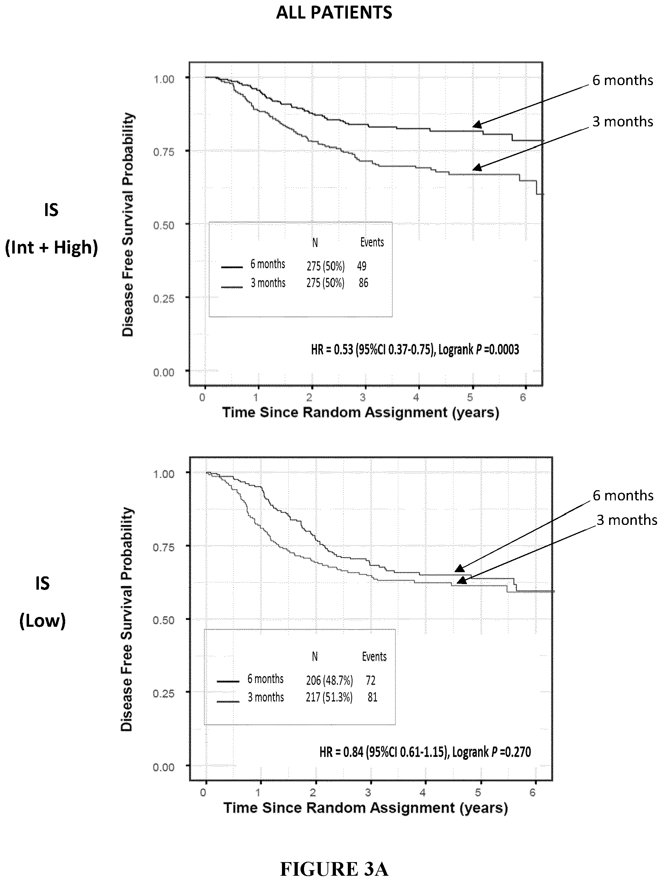

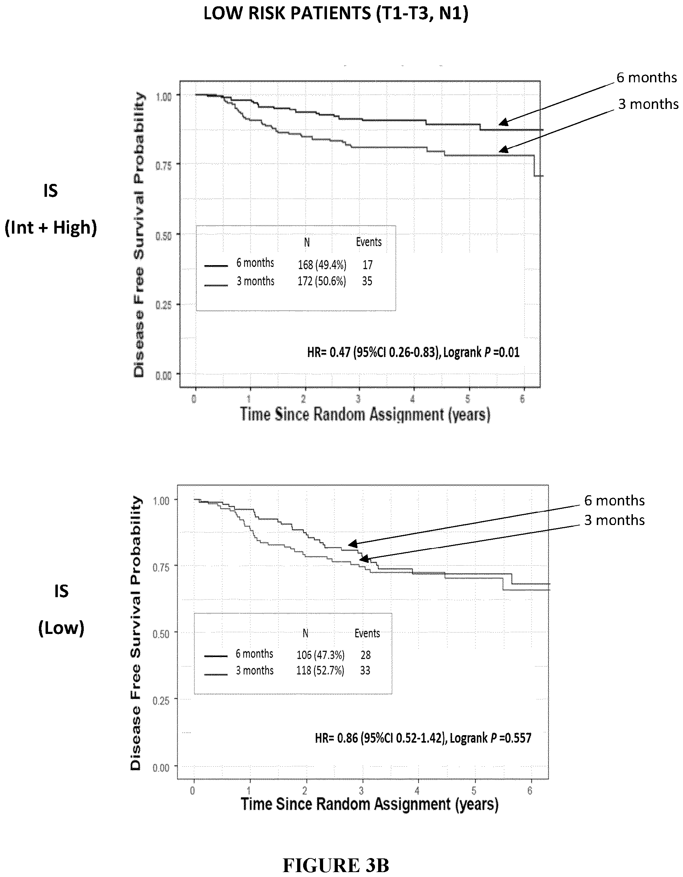

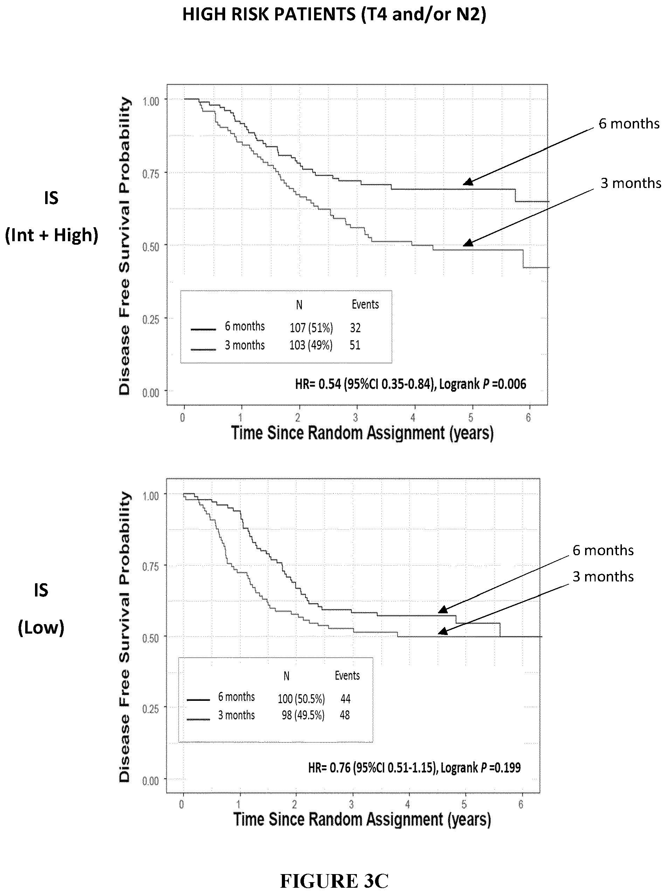

**Figure 3****. Efficacy of 3 months versus 6 months of mFOLFOX6 therapy according to the Immunoscore^{®} status.** (A) A beneficial effect of the 6 months- as compared to 3 months- FOLFOX6 regimen was observed in patients with a CD8 CD3 (Int+High) status (all patients, see top graph). (B, C) This benefit was retained for these patients in the low risk tumors (T1-T3 and N1) and in the high-risk tumors (T4 and/or N2 tumors) (see top graphs). By contrast, no significant benefit of the 6 months-FOLFOX6 regimen was observed for patients with a CD8 CD3 (Low) status, whether the patients were high-risk or low-risk (see bottom graphs). For said patients, moderate benefit of the 6 months-FOLFOX6 regimen was observed in the first 3 years but canceled thereafter.

### Detailed description of the invention

### Cancers

The methods of the invention apply to colorectal cancer.

### Samples

As used herein, the term "tumor tissue sample" means any tissue tumor sample derived from the patient. Said tissue sample is obtained for the purpose of the *in vitro* evaluation. In some embodiments, the tumor sample may result from the tumor resected from the patient. In some embodiments, the tumor sample may result from a biopsy performed in the primary tumor of the patient or performed in metastatic sample distant from the primary tumor of the patient. For example an endoscopical biopsy performed in the bowel of the patient affected by a colorectal cancer. Typically the tumor tissue sample is fixed in formalin and embedded in a rigid fixative, such as paraffin (wax) or epoxy, which is placed in a mould and later hardened to produce a block which is readily cut. Thin slices of material can be then prepared using a microtome, placed on a glass slide and submitted e.g. to immunohistochemistry (using an IHC automate such as BenchMark^{®} XT, for obtaining stained slides). The tumor tissue sample can be used in microarrays, called as tissue microarrays (TMAs). TMA consists of paraffin blocks in which up to 1000 separate tissue cores are assembled in array fashion to allow multiplex histological analysis. This technology allows rapid visualization of molecular targets in tissue specimens at a time, either at the DNA, RNA or protein level. TMA technology is described in WO2004000992, US8068988, Olli et al 2001 Human Molecular Genetics, Tzankov et al 2005, Elsevier; Kononen et al 1198; Nature Medicine.

A tumor tissue sample can encompass (i) a global primary tumor (as a whole), (ii) a tissue sample from the center of the tumor, (iii) a tissue sample from the tissue directly surrounding the tumor which tissue may be more specifically named the "invasive margin" of the tumor, (iv) lymphoid islets in close proximity with the tumor, (v) the lymph nodes located at the closest proximity of the tumor, (vi) a tumor tissue sample collected prior surgery (for follow-up of patients after treatment for example), and (vii) a distant metastasis.

As used herein the "invasive margin" has its general meaning in the art and refers to the cellular environment surrounding the tumor. In some embodiments, the tumor tissue sample, irrespective of whether it is derived from the center of the tumor, from the invasive margin of the tumor, or from the closest lymph nodes, encompasses pieces or slices of tissue that have been removed from the tumor center of from the invasive margin surrounding the tumor, including following a surgical tumor resection or following the collection of a tissue sample for biopsy, for further quantification of one or several biological markers, notably through histology or immunohistochemistry methods, through flow cytometry methods and through methods of gene or protein expression analysis, including genomic and proteomic analysis. The tumor tissue sample can, of course, be subjected to a variety of well-known post-collection preparative and storage techniques (e.g., fixation, storage, freezing, etc.). The sample can be fresh, frozen, fixed (e.g., formalin fixed), or embedded (e.g., paraffin embedded). In some embodiments, when the quantification of the number of tumor-draining lymph nodes is performed in the resected tumor, the tumor tissue sample results from said resected tumor and encompasses the center of the tumor, and optionally the invasive margin of the tumor. In said embodiments, the quantification of the marker of the immune adaptive response is typically performed by immunohistochemistry (IHC) a described after. In some embodiments, when the quantification of the number of tumor-draining lymph nodes is performed is determined by imagery, the tumor tissue sample results from a biopsy. In said embodiments, the quantification of the marker of the immune adaptive response is typically performed by determining the expression level of at least one gene.

A tumor sample may be selected from the group consisting of (i) a global primary tumor (as a whole), (ii) a tissue sample from the center of the tumor, (iii) a tissue sample from the tissue directly surrounding the tumor which tissue may be more specifically named the "invasive margin" of the tumor, (iv) the lymph nodes located at the closest proximity of the tumor or a tertiary lymphoid structure induced by the tumor, (v) a tumor biopsy performed at any time and typically prior surgery, and (vi) a distant metastasis.

The two or more biological markers are herein quantified in the center of the tumor and in the invasive margin of the tumor.

The sample can be fresh, frozen, fixed (e.g., formalin fixed), or embedded (e.g., paraffin embedded). In a particular embodiment the tumor sample results from biopsy performed in a tumor of the patient.

An example is an endoscopical biopsy performed in the bowel of the patient suffering from colorectal cancer or suspected to suffer from colorectal cancer.

### Biological markers

According to the present invention, the methods comprise quantifying include CD3 and CD8 that are expressed by T cells or T cell subsets.

The expression of the CD3 antigen, or the expression of the mRNA thereof is indicative of the level of the adaptive immune response of the patient involving all T lymphocytes and NKT cells. The expression of the CD8 antigen, or the expression of the mRNA thereof, is indicative of the level of the adaptive immune response of the patient involving cytotoxic T lymphocytes.

The methods of the invention may further comprise quantifying at least another biological marker; however quantifying CD3 and CD8 only may be sufficient to achieve the method for determining, modulating or adjusting the treatment regimen according to the invention. As intended herein, a "biological marker" consists of any detectable, measurable and quantifiable parameter that is indicative of the status of the immune response of the cancer patient against the tumor.

Biological markers include the presence of, or the number or density of, cells from the immune system at the tumor site.

Biological markers also include the presence of, or the amount of, proteins specifically produced by cells from the immune system at the tumor site.

Biological markers also include the presence of, or the amount of, any biological material that is indicative of the expression level of genes related to the raising of a specific immune response of the host, at the tumor site. Thus, biological markers include the presence of, or the amount of, messenger RNA (mRNA) transcribed from genomic DNA encoding proteins which are specifically produced by cells from the immune system, at the tumor site.

Biological markers thus include surface antigens that are specifically expressed by cells from the immune system, including by B lymphocytes, T lymphocytes, monocytes/macrophages dendritic cells, NK cells, NKT cells, and NK-DC cells, that are recruited within the tumor tissue or at its close proximity, including within the invasive margin of the tumor and in the closest lymph nodes, or alternatively mRNA encoding for said surface antigens.

Illustratively, proteins used as biological markers also include cytolytic proteins specifically produced by cells from the immune system, like perforin, granulysin and also granzyme-B.

Numerous patent applications have described a large number of biological markers indicative of the status of the immune response which could be used in the methods of the invention.

Typically, one can use the biological markers indicative of the status of the immune response described in WO2015007625, WO2014023706, WO2014009535, WO2013186374, WO2013107907, WO2013107900, WO2012095448, WO2012072750 and WO2007045996.

Typically a combination of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 and 50 distinct biological markers may be quantified, preferably a combination of 2, 3, 4, 5, 6, 7, 8, 9, or 10 biological markers and more preferably a combination of 2, 3, 4, 5, or 6, biological markers.

In a preferred embodiment, the biological markers indicative of the status of the immune response are those described in WO2007045996.

Typically, the biological markers which may be used are the cell density of cells from the immune system.

The methods of the invention comprise quantifying the density of CD3+ cells and the density of CD8+ cells.

The methods may further comprise quantifying the density of CD45RO+ cells, the density of GZM-B+ cells and/or the density of CD45RO+ cells. The density of B-cells may also be measured (see WO2013107900 and WO2013107907). The density of DC cells may also be measured (see WO2013107907).

In a preferred embodiment the density of cells from the immune system are quantified in the center of the tumor and/or in the invasive margin of the tumor.

In a preferred embodiment, the method of the present invention is performed by in situ immunohistochemical detection of protein markers of interest, in particular when separate quantifications of the said markers are performed both in the center of the tumor (CT) and in the invasive margin (IM).

In a preferred embodiment, the method of the present invention is performed by in situ immunohistochemical detection of protein markers of interest or mRNA gene expression of interest, either in the whole tumor or biopsy of the tumor, or in the center of the tumor (CT), or in the invasive margin (IM).

The method comprises quantifying the density of CD3+ cells in center of the tumor, the density of CD8+ cells in the center of the tumor, the density of CD3+ cells in the invasive margin, and the density of CD8+ cells in the invasive margin.

The density may be measured in the "cold spot", i.e., in the regions of the tumor sample where the density is the lowest, or in the 2, 3, 4, 5, 6, 7, 8, 9, 10 "cold spots", corresponding to the 2 to 10 area with the lowest densities.

The density may also be measured in the "hot spot", i.e., in the regions where the density is the highest, or in the 2, 3, 4, 5, 6, 7, 8, 9, 10 "hot spots", corresponding to the 2 to 10 area with the highest densities.

One can also determine the mean density on the whole tumor sample.

Typically, the method disclosed in WO2013/186374 or WO2017/194556 may be used for quantifying the immune cells in the tumor sample.

As used herein, the term "marker" consists of any detectable, measurable or quantifiable parameter that is indicative of the status of the adaptive immune response of the subject. In some embodiments, the marker includes the presence of, or the number or density of, cells from the immune system. In some embodiments, the marker includes the presence of, or the amount of proteins specifically produced by cells from the immune system. In some embodiments, the marker includes the presence of, or the amount of, any biological material that is indicative of the level of genes related to the raising of a specific immune response of the host. Thus, in some embodiments, the marker includes the presence of, or the amount of, messenger RNA (mRNA) transcribed from genomic DNA encoding proteins which are specifically produced by cells from the immune system. In some embodiments, the marker includes surface antigens that are specifically expressed by cells from the immune system, including by B lymphocytes, T lymphocytes, monocytes/macrophages dendritic cells, NK cells, NKT cells, and NK-DC cells or alternatively mRNA encoding for said surface antigens. When performing method of the present invention with more than one biological marker, the number of distinct biological markers that are quantified are usually of less than 100 distinct markers, and in most embodiments of less than 50 distinct markers, still preferably less than 20 distinct markers, still preferably less than 15 distinct markers, still preferably less than 10 distinct markers. The additional biological markers that may be quantified, for being indicative of the status of the immune response, comprise the proteins listed in Table 9 of WO2007045996 which are: 18s, ACE, ACTB, AGTR1, AGTR2, APC, APOA1, ARF1, AXIN1, BAX, BCL2, BCL2L1, CXCR5, BMP2, BRCA1, BTLA, C3, CASP3, CASP9, CCL1, CCL11, CCL13, CCL16, CCL17, CCL18, CCL19, CCL2, CCL20, CCL21, CCL22, CCL23, CCL24, CCL25, CCL26, CCL27, CCL28, CCL3, CCL5, CCL7, CCL8, CCNB1, CCND1, CCNE1, CCR1, CCR10, CCR2, CCR3, CCR4, CCR5, CCR6, CCR7, CCR8, CCR9, CCRL2, CD154, CD19, CD1a, CD2, CD226, CD244, PDCD1LG1, CD28, CD34, CD36, CD38, CD3E, CD3G, CD3Z, CD4, CD40LG, CD5, CD54, CD6, CD68, CD69, CLIP, CD80, CD83, SLAMF5, CD86, CD8A, CDH1, CDH7, CDK2, CDK4, CDKN1A, CDKN1B, CDKN2A, CDKN2B, CEACAM1, COL4A5, CREBBP, CRLF2, CSF1, CSF2, CSF3, CTLA4, CTNNB1, CTSC, CX3CL1, CX3CR1, CXCL1, CXCL10, CXCL11, CXCL12, CXCL13, CXCL14, CXCL16, CXCL2, CXCL3, CXCL5, CXCL6, CXCL9, CXCR3, CXCR4, CXCR6, CYP1A2, CYP7A1, DCC, DCN, DEFA6, DICER1, DKK1, Dok-1, Dok-2, DOK6, DVL1, E2F4, EBI3, ECE1, ECGF1, EDN1, EGF, EGFR, EIF4E, CD105, ENPEP, ERBB2, EREG, FCGR3A, CGR3B, FN1, FOXP3, FYN, FZD1, GAPD, GLI2, GNLY, GOLPH4, GRB2, GSK3B, GSTP1, GUSB, GZMA, GZMB, GZMH, GZMK, HLA-B, HLA-C, HLA-, MA, HLA-DMB, HLA-DOA, HLA-DOB, HLA-DPA1, HLA-DQA2, HLA-DRA, HLX1, HMOX1, HRAS, HSPB3, HUWE1, ICAM1, ICAM-2, ICOS, ID1, ifna1, ifna 17, ifna2, ifna5, ifna6, ifna8, IFNAR1, IFNAR2, IFNG, IFNGR1, IFNGR2, IGF1, IHH, IKBKB, IL10, IL12A, IL12B, IL12RB1, IL12RB2, IL13, IL13RA2, IL15, IL15RA, IL17, IL17R, IL17RB, IL18, IL1A, IL1B, IL1R1, IL2, IL21, IL21R, IL23A, IL23R, IL24, IL27, IL2RA, IL2RB, IL2RG, IL3, IL31RA, IL4, IL4RA, IL5, IL6, IL7, IL7RA, IL8, CXCR1, CXCR2, IL9, IL9R, IRF1, ISGF3G, ITGA4, ITGA7, integrin alpha E (antigen CD103, human mucosal lymphocyte, antigen 1; alpha polypeptide), Gene hCG33203, ITGB3, JAK2, JAK3, KLRB1, KLRC4, KLRF1, KLRG1, KRAS, LAG3, LAIR2, LEF1, LGALS9, LILRB3, LRP2, LTA, SLAMF3, MADCAM1, MADH3, MADH7,MAF, MAP2K1, MDM2, MICA, MICB, MKI67, MMP12, MMP9, MTA1, MTSS1, MYC, MYD88, MYH6, NCAM1, NFATC1, NKG7, NLK, NOS2A, P2X7, PDCD1, PECAM-, CXCL4, PGK1, PIAS1, PIAS2, PIAS3, PIAS4, PLAT, PML, PP1A, CXCL7, PPP2CA, PRF1, PROM1, PSMB5, PTCH, PTGS2, PTP4A3, PTPN6, PTPRC, RAB23, RAC/RHO, RAC2, RAF, RB1, RBL1, REN, Drosha, SELE, SELL, SELP, SERPINE1, SFRP1, SIRP beta 1, SKI, SLAMF1, SLAMF6, SLAMF7, SLAMF8, SMAD2, SMAD4, SMO, SMOH, SMURF1, SOCS1, SOCS2, SOCS3, SOCS4, SOCS5, SOCS6, SOCS7, SOD1, SOD2, SOD3, SOS1, SOX17, CD43, ST14, STAM, STAT1, STAT2, STAT3, STAT4, STAT5A, STAT5B, STAT6, STK36, TAP1, TAP2, TBX21, TCF7, TERT, TFRC, TGFA, TGFB1, TGFBR1, TGFBR2, TIM-3, TLR1, TLR10, TLR2, TLR3, TLR4, TLR5, TLR6, TLR7, TLR8, TLR9, TNF, TNFRSF10A, TNFRSF11A, TNFRSF18, TNFRSF1A, TNFRSF1B, OX-40, TNFRSF5, TNFRSF6, TNFRSF7, TNFRSF8, TNFRSF9, TNFSF10, TNFSF6, TOB1, TP53, TSLP, VCAM1, VEGF, WIF1, WNT1, WNT4, XCL1, XCR1, ZAP70 and ZIC2.

In the present specification, the name of each of the genes of interest refers to the internationally recognized name of the corresponding gene, as found in internationally recognized gene sequences and protein sequences databases, including the database from the HUGO Gene Nomenclature Committee. In the present specification, the name of each of the genes of interest may also refer to the internationally recognized name of the corresponding gene, as found in the internationally recognized gene sequences database Genbank. Through these internationally recognized sequence databases, the nucleic acid to each of the gene of interest described herein may be retrieved by one skilled in the art.

In a preferred embodiment, additional biological markers indicative of the status of the immune response include genes or proteins representative of the adaptive immune response, or genes or proteins representative of the immunosuppressive response.

As used herein the expression "gene representative of the adaptive immune response" refers to any gene that is expressed by a cell that is an actor of the adaptive immune response in the tumor or that contributes to the settlement of the adaptive immune response in the tumor. The adaptive immune response, also called "acquired immune response", comprises antigen-dependent stimulation of T cell subtypes, B cell activation and antibody production. For example cells of the adaptive immune response include but are not limited to cytotoxic T cells, T memory T cells, Th1 and Th2 cells, activated macrophages and activated dendritic cells, NK cells and NKT cells. Accordingly, a gene representative of the adaptive immune response may be typically selected from the cluster of the co-modulated genes for the Th1 adaptive immunity, for the cytotoxic response, or for the memory response, and may encode for a Th1 cell surface marker, an interleukin (or an interleukin receptor), or a chemokine or (a chemokine receptor).

In a particular embodiment, the gene representative of the adaptive immune response is selected from the group consisting of
- the family of chemokines and chemokine receptors consisting of: CXCL13, CXCL9, CCL5, CCR2, CXCL10, CXCL11, CXCR3, CCL2 and CX3CL1,
- the family of cytokines consisting of: II,15,
- the TH1 family consisting of: IFNG, IRF1, STAT1, STAT4 and TBX21
- the family of lymphocytes membrane receptors consisting of: ITGAE, CD3D, CD3E, CD3G, CD8A, CD247, CD69 and ICOS,
- the family of cytotoxic molecules consisting of: GNLY, GZMH, GZMA, GZMB, GZMK, GZMM and PRF1,
and the kinase LTK.

Preferred such genes, or corresponding proteins thereof, are disclosed below:
CCL5, CCR2, CD247, CD3E, CD3G, CD8A, CX3CL1, CXCL11, GZMA, GZMB, GZMH, GZMK, IFNG, IL15, IRF1, ITGAE, PRF1, STAT1, TBX21.

As used herein the expression "gene representative of the immunosuppressive response" refers to any gene that is expressed by a cell that is an actor of the immunosuppressive response in the tumor or that contributes to the settlement of the immunosuppressive response in the tumor. For example, the immunosuppressive response comprises
- co-inhibition of antigen-dependent stimulation of T cell subtypes: genes CD276, CTLA4, PDCD1, CD274, TIM-3 or VTCN1 (B7H4),
- inactivation of macrophages and dendritic cells and inactivation of NK cells: genes TSLP, CD1A, or VEGFA
- expression of cancer stem cell marker, differentiation and/or oncogenesis: PROM1, IHH.
- expression of immunosuppressive proteins produced in the tumour environment: genes PF4, REN, VEGFA.

For example cells of the immunosuppressive response include immature dendritic cells (CD1A), regulatory T cells (Treg cells) and Th17 cells expressing IL17A gene. Accordingly, a gene representative of the adaptive immune response may be typically selected from the group of the co-modulated adaptive immune genes, whereas the immunosuppressive genes, may be representative of the inactivation of immune cells (e.g. dendritic cells) and may contribute to induction of an immunosuppressive response. Genes or corresponding proteins representative of the immunosuppressive response are disclosed below:
CD274, CTLA4, IHH, IL17A, PDCD1, PF4, PROM1, REN, TIM-3, TSLP or VEGFA.

In a preferred embodiment, a gene representative of the adaptive immune response is selected from the group consisting of GNLY, CXCL13, CX3CL1, CXCL9, ITGAE, CCL5, GZMH, IFNG, CCR2, CD3D, CD3E, CD3G, CD8A, CXCL10, CXCL11, GZMA, GZMB, GZMK, GZMM, IL15, IRF1, LTK, PRF1, STAT1, CD69, CD247, ICOS, CXCR3, STAT4, CCL2 and TBX21 and a gene representative of the immunosuppressive response is selected from the group consisting of PF4, REN, VEGFA, TSLP, IL17A, PROM1, IHH, CD1A, CTLA4, PDCD1, CD276, CD274, TIM-3 and VTCN1 (B7H4).

Because some genes are more frequently found significant when combining one adaptive gene and one immunosuppressive gene, the most preferred genes are:
- genes representative of the adaptive immune response: CD3G, CD8A, CCR2 and GZMA,
- genes representative of the immunosuppressive response: REN, IL17A, CTLA4 and PDCD1.

The biological markers indicative of the status of the immune response may comprise the expression level of one or more genes from the group consisting of CCR2, CD3D, CD3E, CD3G, CD8A, CXCL10, CXCL11, GZMA, GZMB, GZMK, GZMM, IL15, IRF1, PRF1, STAT1, CD69, ICOS, CXCR3, STAT4, CCL2, and TBX21;
or GZMH, IFNG, CXCL13, GNLY, LAG3, ITGAE, CCL5, CXCL9, PF4, IL17A, TSLP, REN, IHH, PROM1 and VEGFA.

The methods of the invention may also comprise quantifying the expression level of a miRNA cluster comprising: miR.609, miR.518c, miR.520f, miR.220a, miR.362, miR.29a, miR.660, miR.603, miR.558, miR519b, miR.494, miR.130a, or miR.639, as described in WO2012072750.

### General methods for quantifying biological markers

Any one of the methods known by the one skilled in the art for quantifying cellular types, a protein-type or a nucleic acid-type biological marker encompassed herein may be used for performing the method of the invention. Thus any one of the standard and non-standard (emerging) techniques well known in the art for detecting and quantifying a protein or a nucleic acid in a sample can readily be applied.

Expression of a biological marker as described herein may be assessed by any of a wide variety of well-known methods for detecting expression of a transcribed nucleic acid or protein. Non-limiting examples of such methods include immunological methods for detection of secreted, cell-surface, cytoplasmic, or nuclear proteins, protein purification methods, protein function or activity assays, nucleic acid hybridization methods, nucleic acid reverse transcription methods, and nucleic acid amplification methods.

In one preferred embodiment, expression of a marker is assessed using an antibody (e.g. a radio-labeled, chromophore-labeled, fluorophore-labeled, polymer-backbone-antibody, or enzyme-labeled antibody), an antibody derivative (e.g. an antibody conjugated with a substrate or with the protein or ligand of a protein-ligand pair {e.g. biotin-streptavidin}), or an antibody fragment (e.g. a single-chain antibody, an isolated antibody hypervariable domain, etc.) which binds specifically with a marker protein or fragment thereof, including a marker protein which has undergone all or a portion of its normal post-translational modification.

In certain embodiments, a biological marker, or a set of biological markers, may be quantified with any one of the immunohistochemistry methods known in the art.

Typically, for further analysis, one thin section of the tumor, is firstly incubated with labeled antibodies directed against one biological marker of interest. After washing, the labeled antibodies that are bound to said biological marker of interest are revealed by the appropriate technique, depending of the kind of label is borne by the labeled antibody, e.g. radioactive, fluorescent or enzyme label. Multiple labelling can be performed simultaneously.

Immunohistochemistry typically includes the following steps i) fixing the tumor tissue sample with formalin, ii) embedding said tumor tissue sample in paraffin, iii) cutting said tumor tissue sample into sections for staining, iv) incubating said sections with the binding partner specific for the immune checkpoint protein of interest, v) rinsing said sections, vi) incubating said section with a secondary antibody typically biotinylated and vii) revealing the antigen-antibody complex typically with avidin-biotin-peroxidase complex. Accordingly, the tumor tissue sample is firstly incubated with the binding partners having for the immune checkpoint protein of interest. After washing, the labeled antibodies that are bound to the immune checkpoint protein of interest are revealed by the appropriate technique, depending of the kind of label is borne by the labeled antibody, e.g. radioactive, fluorescent or enzyme label. Multiple labelling can be performed simultaneously. Alternatively, the method of the present invention may use a secondary antibody coupled to an amplification system (to intensify staining signal) and enzymatic molecules. Such coupled secondary antibodies are commercially available, e.g. from Dako, EnVision system. Counterstaining may be used, e.g. Hematoxylin & Eosin, DAPI, Hoechst. Other staining methods may be accomplished using any suitable method or system as would be apparent to one of skill in the art, including automated, semi-automated or manual systems.

For example, one or more labels can be attached to the antibody, thereby permitting detection of the target protein (i.e. the biological markers). Exemplary labels include radioactive isotopes, fluorophores, ligands, chemiluminescent agents, enzymes, and combinations thereof. Non-limiting examples of labels that can be conjugated to primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). A variety of other useful fluorescers and chromophores are described in Stryer L (1968) Science 162:526-533 and Brand L and Gohlke J R (1972) Annu. Rev. Biochem. 41:843-868. Affinity ligands can also be labeled with enzymes (e.g. horseradish peroxidase, alkaline phosphatase, beta-lactamase), radioisotopes (e.g. 3H, 14C, 32P, 35S or 125I) and particles (e.g. gold). The different types of labels can be conjugated to an affinity ligand using various chemistries, e.g. the amine reaction or the thiol reaction. However, other reactive groups than amines and thiols can be used, e.g. aldehydes, carboxylic acids and glutamine. Various enzymatic staining methods are known in the art for detecting a protein of interest. For example, enzymatic interactions can be visualized using different enzymes such as peroxidase, alkaline phosphatase, or different chromogens such as DAB, AEC or Fast Red. In some embodiments, the label is a quantum dot. For example, Quantum dots (Qdots) are becoming increasingly useful in a growing list of applications including immunohistochemistry, flow cytometry, and plate-based assays, and may therefore be used in conjunction with this invention. Qdot nanocrystals have unique optical properties including an extremely bright signal for sensitivity and quantitation; high photostability for imaging and analysis. A single excitation source is needed, and a growing range of conjugates makes them useful in a wide range of cell-based applications. Qdot Bioconjugates are characterized by quantum yields comparable to the brightest traditional dyes available. Additionally, these quantum dot-based fluorophores absorb 10-1000 times more light than traditional dyes. The emission from the underlying Qdot quantum dots is narrow and symmetric which means overlap with other colors is minimized, resulting in minimal bleed through into adjacent detection channels and attenuated crosstalk, in spite of the fact that many more colors can be used simultaneously. In other examples, the antibody can be conjugated to peptides or proteins that can be detected via a labeled binding partner or antibody. In an indirect IHC assay, a secondary antibody or second binding partner is necessary to detect the binding of the first binding partner, as it is not labeled.

In some embodiments, the resulting stained specimens are each imaged using a system for viewing the detectable signal and acquiring an image, such as a digital image of the staining. Methods for image acquisition are well known to one of skill in the art. For example, once the sample has been stained, any optical or non-optical imaging device can be used to detect the stain or biomarker label, such as, for example, upright or inverted optical microscopes, scanning confocal microscopes, cameras, scanning or tunneling electron microscopes, canning probe microscopes and imaging infrared detectors. In some examples, the image can be captured digitally. The obtained images can then be used for quantitatively or semi-quantitatively determining the amount of the immune checkpoint protein in the sample, or the absolute number of cells positive for the maker of interest, or the surface of cells positive for the maker of interest. Various automated sample processing, scanning and analysis systems suitable for use with IHC are available in the art. Such systems can include automated staining and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples. See, e.g., the CAS-200 system (Becton, Dickinson & Co.). In particular, detection can be made manually or by image processing techniques involving computer processors and software. Using such software, for example, the images can be configured, calibrated, standardized and/or validated based on factors including, for example, stain quality or stain intensity, using procedures known to one of skill in the art (see e.g., published U.S. Patent Publication No. US20100136549). The image can be quantitatively or semi-quantitatively analyzed and scored based on staining intensity of the sample. Quantitative or semi-quantitative histochemistry refers to method of scanning and scoring samples that have undergone histochemistry, to identify and quantify the presence of the specified biomarker (i.e. immune checkpoint protein). Quantitative or semi-quantitative methods can employ imaging software to detect staining densities or amount of staining or methods of detecting staining by the human eye, where a trained operator ranks results numerically. For example, images can be quantitatively analyzed using a pixel count algorithms and tissue recognition pattern (e.g. Aperio Spectrum Software, Automated QUantitatative Analysis platform (AQUA^{®} platform), or Tribvn with Ilastic and Calopix software), and other standard methods that measure or quantitate or semi-quantitate the degree of staining; see e.g., U.S. Pat. No. 8,023,714; U.S. Pat. No. 7,257,268; U.S. Pat. No. 7,219,016; U.S. Pat. No. 7,646,905; published U.S. Patent Publication No. US20100136549 and 20110111435; Camp et al. (2002) Nature Medicine, 8:1323-1327; Bacus et al. (1997) Analyt Quant Cytol Histol, 19:316-328). A ratio of strong positive stain (such as brown stain) to the sum of total stained area can be calculated and scored. The amount of the detected biomarker (i.e. the immune checkpoint protein) is quantified and given as a percentage of positive pixels and/or a score. For example, the amount can be quantified as a percentage of positive pixels. In some examples, the amount is quantified as the percentage of area stained, e.g., the percentage of positive pixels. For example, a sample can have at least or about at least or about 0, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 40%, 45%, 50%, 55%, 60%, 65%, 70%, 75%, 80%, 85%, 90%, 95% or more positive pixels as compared to the total staining area. For example, the amount can be quantified as an absolute number of cells positive for the maker of interest. In some embodiments, a score is given to the sample that is a numerical representation of the intensity or amount of the histochemical staining of the sample, and represents the amount of target biomarker (e.g., the immune checkpoint protein) present in the sample. Optical density or percentage area values can be given a scaled score, for example on an integer scale.

Thus, in some embodiments, the method of the present invention comprises the steps consisting in i) providing one or more immunostained slices of tissue section obtained by an automated slide-staining system by using a binding partner capable of selectively interacting with the biological marker, ii) proceeding to digitalisation of the slides of step i) by high resolution scan capture, iii) detecting the slice of tissue section on the digital picture iv) providing a size reference grid with uniformly distributed units having a same surface, said grid being adapted to the size of the tissue section to be analysed, and v) detecting, quantifying and measuring intensity or the absolute number of stained cells in each unit.

Multiplex tissue analysis techniques are particularly useful for quantifying several immune checkpoint proteins in the tumor tissue sample. Such techniques should permit at least five, or at least ten or more biomarkers to be measured from a single tumor tissue sample. Furthermore, it is advantageous for the technique to preserve the localization of the biomarker and be capable of distinguishing the presence of biomarkers in cancerous and non-cancerous cells. Such methods include layered immunohistochemistry (L-IHC), layered expression scanning (LES) or multiplex tissue immunoblotting (MTI) taught, for example, in U.S. Pat. Nos. 6,602,661, 6,969,615, 7,214,477 and 7,838,222; U.S. Publ. No. 2011/0306514; and in Chung & Hewitt, Meth Mol Biol, Prot Blotting Detect, Kurlen & Scofield, eds. 536: 139-148, 2009, each reference teaches making up to 8, up to 9, up to 10, up to 11 or more images of a tissue section on layered and blotted membranes, papers, filters and the like, can be used. Coated membranes useful for conducting the L-IHC/MTI process are available from 20/20 GeneSystems, Inc. (Rockville, MD).

In some embodiments, the L-IHC method can be performed on any of a variety of tissue samples, whether fresh or preserved. The samples included core needle biopsies that were routinely fixed in 10% normal buffered formalin and processed in the pathology department. Standard five µm thick tissue sections were cut from the tissue blocks onto charged slides that were used for L-IHC. Thus, L-IHC enables testing of multiple markers in a tissue section by obtaining copies of molecules transferred from the tissue section to plural bioaffinity- coated membranes to essentially produce copies of tissue "images." In the case of a paraffin section, the tissue section is deparaffinized as known in the art, for example, exposing the section to xylene or a xylene substitute such as NEO-CLEAR^{®}, and graded ethanol solutions. The section can be treated with a proteinase, such as, papain, trypsin, proteinase K and the like. Then, a stack of a membrane substrate comprising, for example, plural sheets of a 10 µm thick coated polymer backbone with 0.4 µm diameter pores to channel tissue molecules, such as, proteins, through the stack, then is placed on the tissue section. The movement of fluid and tissue molecules is configured to be essentially perpendicular to the membrane surface. The sandwich of the section, membranes, spacer papers, absorbent papers, weight and so on can be exposed to heat to facilitate movement of molecules from the tissue into the membrane stack. A portion of the proteins of the tissue are captured on each of the bioaffinity-coated membranes of the stack (available from 20/20 GeneSystems, Inc., Rockville, MD). Thus, each membrane comprises a copy of the tissue and can be probed for a different biomarker using standard immunoblotting techniques, which enables open-ended expansion of a marker profile as performed on a single tissue section. As the amount of protein can be lower on membranes more distal in the stack from the tissue, which can arise, for example, on different amounts of molecules in the tissue sample, different mobility of molecules released from the tissue sample, different binding affinity of the molecules to the membranes, length of transfer and so on, normalization of values, running controls, assessing transferred levels of tissue molecules and the like can be included in the procedure to correct for changes that occur within, between and among membranes and to enable a direct comparison of information within, between and among membranes. Hence, total protein can be determined per membrane using, for example, any means for quantifying protein, such as, biotinylating available molecules, such as, proteins, using a standard reagent and method, and then revealing the bound biotin by exposing the membrane to a labeled avidin or streptavidin; a protein stain, such as, Blot fastStain, Ponceau Red, brilliant blue stains and so on, as known in the art.

In some embodiments, the present methods utilize Multiplex Tissue Imprinting (MTI) technology for measuring biomarkers, wherein the method conserves precious biopsy tissue by allowing multiple biomarkers, in some cases at least six biomarkers.

In some embodiments, alternative multiplex tissue analysis systems exist that may also be employed as part of the present invention. One such technique is the mass spectrometry-based Selected Reaction Monitoring (SRM) assay system ("Liquid Tissue" available from OncoPlexDx (Rockville, MD)). That technique is described in U.S. Pat. No. 7,473,532.

In some embodiments, the method of the present invention utilized the multiplex IHC technique developed by GE Global Research (Niskayuna, NY). That technique is described in U.S. Pub. Nos. 2008/0118916 and 2008/0118934. There, sequential analysis is performed on biological samples containing multiple targets including the steps of binding a fluorescent probe to the sample followed by signal detection, then inactivation of the probe followed by binding probe to another target, detection and inactivation, and continuing this process until all targets have been detected.

In some embodiments, multiplex tissue imaging can be performed when using fluorescence (e.g. fluorophore or Quantum dots) where the signal can be measured with a multispectral imagine system. Multispectral imaging is a technique in which spectroscopic information at each pixel of an image is gathered and the resulting data analyzed with spectral image - processing software. For example, the system can take a series of images at different wavelengths that are electronically and continuously selectable and then utilized with an analysis program designed for handling such data. The system can thus be able to obtain quantitative information from multiple dyes simultaneously, even when the spectra of the dyes are highly overlapping or when they are co-localized, or occurring at the same point in the sample, provided that the spectral curves are different. Many biological materials auto fluoresce, or emit lower- energy light when excited by higher-energy light. This signal can result in lower contrast images and data. High-sensitivity cameras without multispectral imaging capability only increase the autofluorescence signal along with the fluorescence signal. Multispectral imaging can unmix, or separate out, autofluorescence from tissue and, thereby, increase the achievable signal-to-noise ratio. Briefly the quantification can be performed by following steps: i) providing a tumor tissue microarray (TMA) obtained from the patient, ii) TMA samples are then stained with anti-antibodies having specificity of the immune checkpoint protein(s) of interest, iii) the TMA slide is further stained with an epithelial cell marker to assist in automated segmentation of tumour and stroma, iv) the TMA slide is then scanned using a multispectral imaging system, v) the scanned images are processed using an automated image analysis software (e.g.Perkin Elmer Technology) which allows the detection, quantification and segmentation of specific tissues through powerful pattern recognition algorithms. The machine-learning algorithm was typically previously trained to segment tumor from stroma and identify cells labelled.

Determining an expression level of a gene in a tumor sample obtained from a patient can be implemented by a panel of techniques well known in the art.

Typically, an expression level of a gene is assessed by determining the quantity of mRNA produced by this gene.

Methods for determining a quantity of mRNA are well known in the art. For example nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The thus extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous.

Other methods of Amplification include ligase chain reaction (LCR), transcription-mediated amplification (TMA), strand displacement amplification (SDA) and nucleic acid sequence based amplification (NASBA), quantitative new generation sequencing of RNA (NGS).

Nucleic acids (s) comprising at least 10 nucleotides and exhibiting sequence complementarity or homology to the mRNA of interest herein find utility as hybridization probes or amplification primers. It is understood that such nucleic acids need not be completely identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500 nucleotides. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

Nucleic acids which may be used as primers or probes in the above amplification and detection method may be assembled as a kit. Such a kit includes consensus primers and molecular probes. A preferred kit also includes the components necessary to determine if amplification has occurred. A kit may also include, for example, PCR buffers and enzymes; positive control sequences, reaction control primers; and instructions for amplifying and detecting the specific sequences.

In a particular embodiment, the expression of a biological marker as described herein may be assessed by tagging the biomarker (in its DNA, RNA or protein for) with a digital oligonucleotide barcode, and to measure or count the number of barcodes.

In a particular embodiment, the methods of the invention comprise the steps of providing total RNAs extracted from cumulus cells and subjecting the RNAs to amplification and hybridization to specific probes, more particularly by means of a quantitative or semi-quantitative RT-PCR.

Probes made using the disclosed methods can be used for nucleic acid detection, such as in situ hybridization (ISH) procedures (for example, fluorescence in situ hybridization (FISH), chromogenic in situ hybridization (CISH) and silver in situ hybridization (SISH)) or comparative genomic hybridization (CGH).

In situ hybridization (ISH) involves contacting a sample containing target nucleic acid sequence (e.g., genomic target nucleic acid sequence) in the context of a metaphase or interphase chromosome preparation (such as a cell or tissue sample mounted on a slide) with a labeled probe specifically hybridizable or specific for the target nucleic acid sequence (e.g., genomic target nucleic acid sequence). The slides are optionally pretreated, e.g., to remove paraffin or other materials that can interfere with uniform hybridization. The sample and the probe are both treated, for example by heating to denature the double stranded nucleic acids. The probe (formulated in a suitable hybridization buffer) and the sample are combined, under conditions and for sufficient time to permit hybridization to occur (typically to reach equilibrium). The chromosome preparation is washed to remove excess probe, and detection of specific labeling of the chromosome target is performed using standard techniques.

For example, a biotinylated probe can be detected using fluorescein-labeled avidin or avidin-alkaline phosphatase. For fluorochrome detection, the fluorochrome can be detected directly, or the samples can be incubated, for example, with fluorescein isothiocyanate (FITC)-conjugated avidin. Amplification of the FITC signal can be conducted, if necessary, by incubation with biotin-conjugated goat antiavidin antibodies, washing and a second incubation with FITC-conjugated avidin. For detection by enzyme activity, samples can be incubated, for example, with streptavidin, washed, incubated with biotin-conjugated alkaline phosphatase, washed again and pre-equilibrated (e.g., in alkaline phosphatase (AP) buffer). For a general description of in situ hybridization procedures, see, e.g., U.S. Pat. No. 4,888,278.

Numerous procedures for FISH, CISH, and SISH are known in the art. For example, procedures for performing FISH are described in U.S. Pat. Nos. 5,447,841; 5,472,842; and 5,427,932; and for example, in Pinkel et al., Proc. Natl. Acad. Sci. 83:2934-2938, 1986; Pinkel et al., Proc. Natl. Acad. Sci. 85:9138-9142, 1988; and Lichter et al., Proc. Natl. Acad. Sci. 85:9664-9668, 1988. CISH is described in, e.g., Tanner et al., Am. J. Pathol. 157:1467-1472, 2000 and U.S. Pat. No. 6,942,970. Additional detection methods are provided in U.S. Pat. No. 6,280,929.

Numerous reagents and detection schemes can be employed in conjunction with FISH, CISH, and SISH procedures to improve sensitivity, resolution, or other desirable properties. As discussed above probes labeled with fluorophores (including fluorescent dyes and QUANTUM DOTS^{®}) can be directly optically detected when performing FISH. Alternatively, the probe can be labeled with a nonfluorescent molecule, such as a hapten (such as the following non-limiting examples: biotin, digoxigenin, DNP, and various oxazoles, pyrrazoles, thiazoles, nitroaryls, benzofurazans, triterpenes, ureas, thioureas, rotenones, coumarin, courmarin-based compounds, Podophyllotoxin, Podophyllotoxin-based compounds, and combinations thereof), ligand or other indirectly detectable moiety. Probes labeled with such non-fluorescent molecules (and the target nucleic acid sequences to which they bind) can then be detected by contacting the sample (e.g., the cell or tissue sample to which the probe is bound) with a labeled detection reagent, such as an antibody (or receptor, or other specific binding partner) specific for the chosen hapten or ligand. The detection reagent can be labeled with a fluorophore (e.g., QUANTUM DOT^{®}) or with another indirectly detectable moiety, or can be contacted with one or more additional specific binding agents (e.g., secondary or specific antibodies), which can be labeled with a fluorophore.

In other examples, the probe, or specific binding agent (such as an antibody, e.g., a primary antibody, receptor or other binding agent) is labeled with an enzyme that is capable of converting a fluorogenic or chromogenic composition into a detectable fluorescent, colored or otherwise detectable signal (e.g., as in deposition of detectable metal particles in SISH). As indicated above, the enzyme can be attached directly or indirectly via a linker to the relevant probe or detection reagent. Examples of suitable reagents (e.g., binding reagents) and chemistries (e.g., linker and attachment chemistries) are described in U.S. Patent Application Publications Nos. 2006/0246524; 2006/0246523, and 2007/0117153.

It will be appreciated by those of skill in the art that by appropriately selecting labelled probe-specific binding agent pairs, multiplex detection schemes can be produced to facilitate detection of multiple target nucleic acid sequences (e.g., genomic target nucleic acid sequences) in a single assay (e.g., on a single cell or tissue sample or on more than one cell or tissue sample). For example, a first probe that corresponds to a first target sequence can be labelled with a first hapten, such as biotin, while a second probe that corresponds to a second target sequence can be labelled with a second hapten, such as DNP. Following exposure of the sample to the probes, the bound probes can be detected by contacting the sample with a first specific binding agent (in this case avidin labelled with a first fluorophore, for example, a first spectrally distinct QUANTUM DOT^{®}, e.g., that emits at 585 nm) and a second specific binding agent (in this case an anti-DNP antibody, or antibody fragment, labelled with a second fluorophore (for example, a second spectrally distinct QUANTUM DOT^{®}, e.g., that emits at 705 nm). Additional probes/binding agent pairs can be added to the multiplex detection scheme using other spectrally distinct fluorophores. Numerous variations of direct, and indirect (one step, two step or more) can be envisioned, all of which are suitable in the context of the disclosed probes and assays.

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In a particular embodiment, the methods of the invention comprise the steps of providing total RNAs extracted from cumulus cells and subjecting the RNAs to amplification and hybridization to specific probes, more particularly by means of a quantitative or semi-quantitative RT-PCR.

In another preferred embodiment, the expression level is determined by DNA chip analysis. Such DNA chip or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To determine the expression level, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210).

The expression level of a gene may be expressed as absolute expression level or normalized expression level. Both types of values may be used in the present method. The expression level of a gene is preferably expressed as normalized expression level when quantitative PCR is used as method of assessment of the expression level because small differences at the beginning of an experiment could provide huge differences after a number of cycles.

Typically, expression levels are normalized by correcting the absolute expression level of a gene by comparing its expression to the expression of a gene that is not relevant for determining the cancer stage of the patient, e.g., a housekeeping gene that is constitutively expressed. Suitable genes for normalization include housekeeping genes such as the actin gene ACTB, ribosomal 18S gene, GUSB, PGK1 and TFRC. This normalization allows comparing the expression level of one sample, e.g., a patient sample, with the expression level of another sample, or comparing samples from different sources.

### Therapeutic agents

It is herein provided a method for determining, modulating or adjusting a treatment regimen with a chemotherapeutic agent in a patient affected with a cancer, wherein said agent is able to leverage or promote a tumor-targeted immune response, as defined in the claims. The methods of the invention do not encompass treatment *per se,* rather treatment decisions.

The treatment may consist of an adjuvant therapy (i.e. treatment after chirurgical resection of the primary tumor) of a neoadjuvant therapy (i.e. treatment before chirurgical resection of the primary tumor).

The term "chemotherapeutic agent" refers to chemical compounds that are effective in inhibiting tumor growth.

As described in Galluzzi et al, 2016, Cancer Immunol Res, 4(11): 895-902, and Galluzzi et al, 2015, Cancer Cell Review, 28(6): 690-714, agents that are enable to cause tumor-targeting immune responses, either increase the immunogenicity (antigenicity or adjuvanticity) of malignant cells ("on-target" immunostimulation), or interact with immune effectors or immunosuppressive cell populations ("off-target" immunostimulation).

In a preferred embodiment, the chemotherapeutic agent is able to cause immunologic cell death (ICD). Unlike normal apoptosis, which is mostly nonimmunogenic or even tolerogenic, immunogenic apoptosis of cancer cells can induce an effective antitumor immune response through activation of dendritic cells (DCs) and consequent activation of specific T cell response.

The chemotherapeutic agent is oxaliplatin, used either alone or in combination with 5-fluorouracil (5FU) and/or capecitabine.

In a particular embodiment, the therapy is FOLFOX (oxaliplatin+5FU), mFOLFOX6 (oxaliplatin+5FU+leucovorin) or CAPOX (oxaliplatin+capecitabine).

Alternatively, chemotherapeutic agents other than fluoropyrimidines (such as 5-Fluorouracil or capecitabine), that are able to leverage or promote a tumor-targeted immune response, include, without limitation, the following:
Bleomycin
Bortezomib
Alkylating agents (such as Cyclophosphamide)
Dacarbazine
Taxoids (such as Docetaxel or Paclitaxel)
Anthracyclins, such as Doxorubicin
Irinotecan
Gemcitabine
Idarubicine
Melphalan
Pemetrexed
Vinorelbine
Chemotherapeutic agent able to induce ICD are platinum, or a platinum salt, derivative or analog, including oxaliplatin, cisplatin and carboplatin, as well as bleomycin, bortezomib, cyclophosphamide, or anthracyclins, such as doxorubicin.

In a particular embodiment, the chemotherapeutic agent can be combined with another therapeutic agent, such as an immunotherapeutic agent (e.g. an antibody).

### Reference values

Predetermined reference values used for comparison may comprise "cut-off" or "threshold" values that may be determined as described herein. Each reference ("cut-off") value for each gene of interest may be predetermined by carrying out a method comprising the steps of
a) providing a collection of tumor tissue samples from patients suffering of cancer;
b) determining the expression level of the gene or protein for each tumor tissue sample contained in the collection provided at step a);
c) ranking the tumor tissue samples according to said expression level
d) classifying said tumor tissue samples in pairs of subsets of increasing, respectively decreasing, number of members ranked according to their expression level,
e) providing, for each tumor tissue sample provided at step a), information relating to the actual clinical outcome for the corresponding cancer patient (i.e. the duration of the disease-free survival (DFS) or the overall survival (OS) or the time to recurrence (TTR) or both);
f) for each pair of subsets of tumor tissue samples, obtaining a Kaplan Meier percentage of survival curve;
g) for each pair of subsets of tumor tissue samples calculating the statistical significance (p value) between both subsets
h) selecting as reference value for the expression level, the value of expression level for which the p value is the smallest.

For example the expression level of a gene X has been assessed for 100 cancer samples of 100 patients. The 100 samples are ranked according to their expression level. Sample 1 has the best expression level and sample 100 has the worst expression level. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding cancer patient, Kaplan Meier curves are prepared for each of the 99 groups of two subsets. Also for each of the 99 groups, the p value between both subsets was calculated.

The reference value is selected such as the discrimination based on the criterion of the minimum p value is the strongest. In other terms, the expression level corresponding to the boundary between both subsets for which the p value is minimum is considered as the reference value. It should be noted that the reference value is not necessarily the median value of expression levels.

In routine work, the reference value (cut-off value) may be used in the present method to discriminate tumour samples and therefore the corresponding patients.

Kaplan-Meier curves of percentage of survival as a function of time are commonly to measure the fraction of patients living for a certain amount of time after treatment and are well known by the man skilled in the art.

The man skilled in the art also understands that the same technique of assessment of the expression level of a gene should of course be used for obtaining the reference value and thereafter for assessment of the expression level of a gene of a patient subjected to the method of the invention.

Such predetermined reference values of expression level may be determined for any gene defined herein.

As described, e.g. in International patent application WO2017/194556, the reference value may be a distribution of values obtained for each of CD3 and CD8 from a reference group of patients suffering from said cancer. The method of the invention then comprises determining the percentile of the distribution to which the values, obtained when quantifying each of CD3 and CD8, correspond; calculating the arithmetic mean value or the median value of percentile; and
comparing the arithmetic mean value or the median value of percentile obtained with a predetermined reference arithmetic mean value or a predetermined median value of percentile.

When the patient is a patient with colorectal cancer, predetermined reference values may be as described in Pagès et al, Lancet 2018; 391: 2128-2139. An international consortium of 14 centres in 13 countries, led by the Society for Immunotherapy of Cancer, assessed the Immunoscore assay in patients with TNM stage I-III colon cancer. Patients were randomly assigned to a training set, an internal validation set, or an external validation set. Paraffin sections of the colon tumor and invasive margin from each patient were processed by immunohistochemistry, and the densities of CD3+ and cytotoxic CD8+ T cells in the tumor and in the invasive margin were quantified by digital pathology. An Immunoscore for each patient was derived from the mean of four density percentiles. The primary endpoint was to evaluate the prognostic value of the Immunoscore for time to recurrence, defined as time from surgery to disease recurrence. Stratified multivariable Cox models were used to assess the associations between Immunoscore and outcomes, adjusting for potential confounders. Harrell's C-statistics was used to assess model performance.

The immune status of the patient, referred to as the "Immunoscore (IS)" in the Examples, can be classified in three groups (Low, Intermediate, High), as follows:

A mean percentile of the 4 individual percentiles of the 4 markers (CD3ct, CD8ct, CD3im, CD8im) is determined, corresponding to the density of CD3 cells in CT and IM regions and the density of CD8 cells in CT and IM regions.
- Low IS corresponds to a mean percentile below 25%,
- Intermediate IS corresponds to a mean percentile between 25% and 70%,
- High IS corresponds to a mean percentile above 70%.

### Methods for modulating the treatment regimen

According to conventional chemotherapy regimens, anticancer drugs are administered in cycles near or at the Maximum Tolerated Dose, that can alternate with long drug-free periods to allow patient's recovery from adverse drug reactions.

For example, typical colon cancer chemotherapies are capecitabine, LV5FU2, CAPOX, FOLFOX4 or mFOLFOX6, preferably administered according to the following regimens:
- Capecitabine: 1250 mg/m² capecitabine administered twice a day, for 14 days (duration of the cycle), cycle to be repeated every 3 weeks;
- LV5FU2: 400 mg/m² AF (folinic acid) combined with 400 mg/m² 5-FUb (5-fluorouracil, bolus) and 2400 5-FUc (5-fluorouracil, continued), administered for 46h (duration of the cycle), cycle to be repeated every 2 weeks;
- CAPOX: 130 mg/m² oxaliplatin administered the first day (day 1) of the cycle, combined with 1000 mg/m² capecitabine administered twice a day from day 1 for 14 days (duration of the cycle), cycle to be repeated every 3 weeks;
- FOLFOX4: 85 mg/m² oxaliplatin, 400 mg/m² AF, 400 mg/m² 5-FUb, and 600 mg/m² 5-FUc, administered on day 1, followed by 400 mg/m²AF, 400 mg/m² 5-FUb, 600 mg/ m² 5-FUc administered on day 2 (duration of the cycle: 2 days), cycle to be repeated every two weeks;
- mFOLFOX6: 85 mg/m² oxaliplatin, 400 mg/m² AF, 400 mg/m² 5-FUb, 2400 mg/m² 5-FUc, administered for 46h (duration of the cycle), cycle to be repeated every 2 weeks.

Based on the *in vitro* methods of the invention, the skilled person may herein determine, modulate or adjust a treatment regimen, so that the patient is more likely to benefit from said treatment.

The dosage, the frequency of administration (namely the number of cycles), and/or the duration of treatment may be adapted accordingly.

Is disclosed herein, but not claimed, a method for determining sensitivity of a patient affected with a cancer, toward a treatment with a chemotherapeutic agent, wherein said agent is able to leverage or promote a tumor-targeted immune response, preferably by causing immunological cell death (ICD), which method comprises quantifying at least two biological markers which are CD8 and CD3 in a tumor sample from the patient.

Is disclosed herein, but not claimed, methods of treating a patient suffering from cancer, which method comprises quantifying at least two biological markers which are CD8 and CD3 in a tumor sample from the patient, comparing the values obtained in a) to predetermined reference values, and c)
- when the values obtained in a) are superior to a predetermined reference value, adjusting the dose, duration of treatment and/or frequency of administration compared to a standard treatment reference, of a chemotherapeutic agent in a patient affected with a cancer, wherein said agent is able to leverage or promote a tumor-targeted immune response; or
- treating the patient with a therapeutic regimen that does not comprise said chemotherapeutic agent.

A "cumulative dose" refers to the quantity of agent that is administered to the patient over the period of treatment.

The cumulative dose may be modulated by playing on one or several parameters, such as the frequency of administration (e.g. the time period between two cycles of administration), the duration of cycles of administration, and/or the dosage for each administration.

In a particular embodiment, it is contemplated to modulate the chemotherapy by implementing a metronomic protocol. A "metronomic chemotherapy" is the frequent administration of chemotherapy agents at doses below the Maximum Tolerated Dose (MTD) and with no prolonged drug-free break.

Alternatively, the cycles of treatment may be repeated with a predetermined frequency (e.g. between 5 days and 15 days, preferably between 5 and 10 days). The duration of the treatment may also prolonged (as proposed in the Experimental Section).

A particular example is set forth below.

A typical regimen when administering oxaliplatin-based chemotherapy is a cyclic administration (during 1 to 3 days, every two or three weeks), leading to a cumulative dose as defined in the claims. The method of the invention makes it possible to provide a benefit for patients who show an intermediate or high IS when treated with said cumulative dose over a course of 6 months.

The chemotherapeutic agents may be administered by any convenient route, preferably by i.v. route. When metronomic chemotherapy is contemplated, oral route may be preferred.

The Examples and Figures illustrate the invention without limiting its scope.

### EXAMPLES

**Abbreviations:** mFOLFOX6: modified FOLFOX6 therapy (infusional 5-fluorouracil plus leucovorin plus oxaliplatin); CAPOX: therapy with capecitadine plus oxaliplatin; IS: Immunoscore^{®} status corresponding to CD8 and CD3 cellular density; High: high IS value; Low: high IS value; Int: Intermediate IS value; IM: Invasive Margin; CT: Core of the Tumor; MSI: microsatellite instability; DFS: disease-free survival; RMST: restricted mean survival time; TNM classification: Tumor, lymph Nodes and Metastasis cancer stage as defined by the American Joint Cancer Committee (Green FL, Page D, Fleming ID: American Joint Cancer Committee Staging Handbook, 6th edition, New York, NY Springer, 2002); T: primary tumor stage (e.g. TX, T0, T1 to T4) ; N: N regional lymph nodes (e.g. NX, N0, N1 to N4); M: metastasis stage (e.g. MX, M0, M1).

### 1. MATERIAL and METHODS

1322 patients suffering from stage III colon cancer (CC), and intended to receive mFOLFOX6 and/or CAPOX therapy over 3 or 6 months, were included in the present analysis.

Tumor samples were collected for each patient before treatment, every two weeks during treatment, and then every 6 months for 5 years.

As described in André et al, 2018, J Clin Oncol 36.1-11, in the 6 months period, the median oxaliplatin (with mFOLFOX6) dose received per patient was 732.39 mg/m² (mean: 8.9 cycles).

In contrast, in the 3 months period, the median oxaliplatin dose received per patient was 494.22 mg/m² (with mFOLFOX6), or 504.44 mg/ m² (with CAPOX).

One formalin-Fixed Paraffin-Embedded (FFPE) block per patient was selected in a central pathology laboratory (Hôpital Ambroise Paré, Boulogne, FR) according to the Immunoscore^{®} Sample Preparation Instructions. Briefly, either one FFPE block or four unstained labeled slides prepared from 4µm-thick adjacent FFPE sections were sent at room temperature to two laboratories (HalioDx , Marseille, France; Immunomonitoring Platform, Hôpital Furopéen Georges Pompidou AP-HP, INSERM, Paris, France) where Immunoscore^{®} testing (Immunohistochemistry (IHC) and image analysis), i.e. measure of CD3 and CD8 expression, was performed blinded to clinical data. Automatized Immunohistochemistry on BenchMark XT (Ventana) was performed within 4 months of sectioning. Sections were incubated at 37°C with primary antibodies: rabbit monoclonal anti-human CD3 (clone HDx2 HalioDx, Marseille, FR) and mouse monoclonal anti-human CD8 (clone HDx1 HalioDx, Marseille, FR) as recommended by the manufacturer. Counterstained slides were digitalized at 20x magnification and 0.45 µm/pixel resolution on a NanoZoomer XR (Hamamatsu).

Image analysis software was used for automatic tissue detection (tumor, healthy non-epithelial tissue and epithelium) and to quantify the density of stained immune CD3+ and CD8+ cells by number of cells per mm2 in both the core of the tumor (CT) and the invasive margin (IM) using a software algorithm (Immunoscore^{®} Analyzer, HalioDx, Marseille, FR) integrated into an image analysis system (Pagès et al, Lancet 2018; 391: 2128-2139; Hermitte et al, 2016 ; 4: 57). IM, defined as a region of 360µm width on each side of the frontier between malignant cells and peritumoral stroma was generated automatically. CD3+ and CD8+ T-cell densities were converted into an Immunoscore^{®} with pre-defined cutoffs, per an algorithm previously developed on patients with stages I to III colon cancer (Pagès et al, supra; Hermitte et al, 2016 ; 4: 57). Immunoscore^{®} utilizes standardized percentile values (0 to 100%) and the algorithm categorizes the continuous Immunoscore^{®} into 5 groups using predefined density scores with a mean percentile of [0-10%], [>10-25%], [>25-70%], [>70-95%] and [>95-100%] corresponding to IS 0, IS 1, IS 2, IS 3, and IS 4 respectively. The 3-group classification corresponds to IS 0-1, IS 2 and IS 3-4 being Immunoscore^{®} Low, Intermediate and High respectively and the categorization into 2 groups corresponds to IS 0-1 (mean percentile 0-25%) and IS 2-3-4 (mean percentile >25-100%) being Immunoscore^{®} Low and High respectively.

Samples were not eligible in case of FFPE block issue, sample identification issue, if the tissue was torn or folded, in case of high IHC staining background, or if a tumor region (CT or IM) was missing. For Immunoscore^{®} analysis quality control, samples were excluded from the analysis if CD3 or CD8 counts were missing from a tumor region (CT or IM) or if staining intensity was deemed low.

### 2. RESULTS

### 2.1. Patient characteristics and IS determination

A total of 1322 patients out of 2010 suffering from stage III colon cancer (CC) with available tumor samples before and throughout treatment were included in the analysis. As compared to the entire population of patients, a lower proportion of low-risk patients (T1-T3N1) and especially T1-stage tumors were observed in patients with sample available for Immunoscore^{®} characterization **(Table 1).**

**Table 1. Multivariate analysis for DFS combining IS and histopathological classifications.**

| | | N | events | HR | 95%Cl | P-value |
|---|---|---|---|---|---|---|
| **Immunoscore classification** | | | | | | |
| | High | 100 | 17 | 1 | | |
| | intermediate | 499 | 133 | 1.745 | 1.053 to 2.892 | |
| | Low | 463 | 167 | 2.321 | 1.409 to 3.824 | **0.0008** |

| **Histopathological classification** | | | | | | |
|---|---|---|---|---|---|---|
| | Low risk (T1-3, N1) | 622 | 129 | 1 | | |
| | High risk (T4 or N2) | 439 | 188 | 2.343 | 1.871 to 2.935 | **<.0001** |

Among the available samples, 82 samples were excluded due to pre-analytical nonconformity. A dedicated software monitored the CD3 and CD8 staining intensities and determined the IS **(****Figure 1A****).** This procedure providing an internal quality control, allowed consistency for the counting of stained cells (Pagès et al 2018, supra) and reproducibility of IS between the two centers involved in IS assessment **(****Figure 1B****).** Overall, 1062 cases (85.6%) reached the quality control.

After translation of the immune densities into a 2-category IS scoring system **(****Figure 1C****),** IS Low and Int+High were observed in n=599 (43.6%) and n=463 (56.4% including 47% Int and 9.4% High) patients, respectively.

### 2.2. IS associated prognostic value

IS in 2 categories was significantly correlated with T stage, T/N stage (T1-3 and N1 *versus* T4 and/or N2), and microsatellite instability (MSI) status.

The primary objective of study was met since a significant difference of the DFS among patient's groups stratified by IS in two categories (Low vs Int+High) in the patients' population was evidenced by univariate analysis (HR= 1.54 (95%CI 1.24-1.93); P=0.0001), and illustrated by Kaplan-Meier curves **(Table 3** and **Figure 2A****).** The 3-year DFS rates were 66.80% [95%CI 62.23-70.95] and 77.14% [95%CI 73.50-80.35] for IS Low and IS Int+High, respectively. IS as a continuous variable **(Table 2)** was also significantly associated with DFS (*P*<0.0001).

**Table 2. Final multivariate model for DFS prediction (with IS in its continuous form)**

| | | **Multivariate analysis (N=999 N events = 296)** | | |
|---|---|---|---|---|
| | | **HR** | **95%Cl** | **P-value** |
| **Immunoscore*** | | 0.988 | 0.982 to 0.993 | <.0001 |
| **Gender*** | | | | |
| | Female | 1 | | |
| | Male | 1.279 | 1.010 to 1.621 | 0.0413 |

| **Tumor/Node Stage** | | | | |
|---|---|---|---|---|
| | T1-2-3 a nd N1 | 1 | | |
| | T4 and/or N2 | 1.949 | 1.513 to 2.510 | <.0001 |
| | Missing | | | |

| **LNR*** | | | | |
|---|---|---|---|---|
| | <=0.3 | 1 | | |
| | >0.3 | 1.932 | 1.451 to 2,571 | <.0001 |

| **Treatment Arm** | | | | |
|---|---|---|---|---|
| | 3M | 1 | | |
| | 6M | 0.711 | 0.564 to 0.895 | 0.0038 |

| | **Microsatellite Instability status** | | | |
|---|---|---|---|---|
| | pMMR | 1 | | |
| | dMMR | 0.564 | 0.309 to 1.033 | 0.0639 |
| | Missing | | | |

In accordance, IS stratified in 3- categories **(****Figure 2B****)** and in 5-categories further discriminate patient's outcome for DFS times (Logrank tests, all *P* <0.001). With IS stratified in 3- categories, a 3-year DFS rate of 85 % was observed in patients with IS High, as compared to 67% for patients with IS Low. With IS stratified in 5- categories, patients with the lowest IS (ISO) presented with a 3-year DFS rate of 55 % as compared to 100% (no relapse or death) in the group with the highest IS (IS4).

Finally, the restricted mean survival time (RMST) was further tested as an alternative measure of the survival time distribution according to IS. RMST was significant in DFS analysis for IS High *vs* IS Low subgroups, similarly to Logrank P-values and HR analyses. For example, RMST for relapse or death events (DFS) showed a gain of 367 days for patients with Immunoscore-High compared to patients with Immunoscore-Low.

In multivariable analysis, IS remained significantly and independently associated with DFS when combined with gender, histological grade, T stage, N stage and MSI status **(Table 1).** Moreover, IS remained independently associated with DFS when combined with T/N stage categorization (high-risk T4 and/or N2; low risk T1-3 and N1). The addition of IS to the T/N stage significantly improved the model discrimination capacity [bootstrap C index mean difference, 0.022; 95%CI 0.005-0.04].

### 2.3. Prognostic value of IS according to T/N stage subgroups

A significant correlation was found between the IS in 2, 3 or 5 categories and the occurrence of relapse or death in the subgroup of patients with low risk T1-3 and N1 tumor (Logrank tests) all *P*<= 0.001) (see **Figure 3**).

In patients with high-risk T4 and/or N2 tumors, IS categorization did not reach significance but the highest IS was constantly observed in patients with the lowest risk of event and the risk of recurrence or death gradually increased along with the decrease of IS **(****Figure 2****).)**

### 2.4. Predictive value of IS for duration of adjuvant FOLFOX6 chemotherapy according to the invention

492 and 481 patients receiving mFOLFOX6 in the 3- and 6-month arms, respectively, were analysed. High-risk tumors (T4 and/or N2 tumors) represented 38.4% (n=408 patients) of the cohort.

A beneficial effect of the 6 months- as compared to 3 months- FOLFOX6 regimen was observed in patients with a (Int+ High) IS (HR = 0.528; 95%CI 0.372 to 0.750; Logrank *P* = 0.0004, **Figure 3A**). This benefit was retained in the low risk tumors (T1-T3 and N1; HR = 0.467, Logrank *P* = 0.010) and in the high-risk tumors (T4 and/or N2 tumors; HR = 0.542, Logrank *P* = 0.0067) (**Figure 3A**).

Strikingly, no significant benefit of the 6 months- FOLFOX6 regimen was observed for patients with a Low IS (HR = 0836, Logrank *P* = 0.269; **(****Figure 3B****).** In those patients, a moderate benefit of the 6 months-FOLFOX6 regimen was observed in the first 3 years but canceled thereafter. This trend was more pronounced in the group of high-risk tumors **(****Figure 3B****).** To further investigate the relationship between density of immune infiltrate and the benefit of 6-months chemotherapy, IS was evaluated as a continuous variable. The clinical benefit of 6 months of chemotherapy, as evaluated with the DFS rate at 3 year (%), becomes more important the higher the IS gets.

To further investigate the relationship between the density of immune infiltrate and the benefit of 6 months chemotherapy, IS was stratified into 5-categories. The results show that the population that benefit the most of a 6 months-chemotherapy are the patients with an Int IS.

### 3. DISCUSSION

The Immunoscore^{®} (IS) test has previously been shown to prognostically classify Stage I-III colon cancer (CC) patients. This test has herein been assessed in a cancer France cohort study, to study 3 versus 6 months of oxaliplatin-based adjuvant chemotherapy in colon cancer patients at various cancer stages (TNM stages T1-T3, N1, T4, N2).

Densities of CD3+ and cytotoxic CD8+ T cells in the tumor and invasive margin of each patient were quantified by digital pathology and converted to Immunoscore^{®} status (High, Low, Intermediate) depending on the measured CD3 and CD8 densities, and using pre-defined published cut-off. The performance of **Immunoscore^{®}** to predict disease-free survival (DFS) was assessed in the modified intention-to-treat population, in each study arm, and was adjusted with relevant clinical features in multivariable Cox models. Harrell's C-statistics was used to investigate the IS performance.

IS was successfully analyzed in 1062 (85.6%) eligible patients. In a 2-category IS analysis, Low and Int+High IS were observed in n=599 (43.6%) and n=463 (56.4%) patients, respectively. IS was significantly correlated with T stage, T/N stage (T1-3 and N1 versus T4 and/or N2), and microsatellite instability status. This study thus validates that Low IS identifies patients with higher-risk of relapse or death [HR=1.54; 95%CI 1.24-1.93, p=0.0001]. The 3-year DFS rates were 66.80% [95%CI 62.23-70.95] and 77.14% [95%CI 73.50-80.35] for Low IS and Int+High IS, respectively. In multivariable analysis, IS remained independently associated with DFS (p<0.0012) when combined with T/N stage. The addition of IS to the T/N stage significantly improved the model discrimination capacity [bootstrap C index mean difference, 0.022; 95%CI 0.005-0.04]. In addition, IS in 3 categories (Low, Int, High) and as a continuous variable were also both significantly associated with DFS (all p<0.001). In univariable analysis, IS was also associated with DFS in 6 months arm (p<0.0001); a similar trend was observed in 3 months arm (p=0.09). The present study demonstrates that only patients with an Intermediate or High IS status did benefit from 6 months of mFOLFOX6 therapy compared with 3 months, both in low (T1-T3, N1) and high-risk (T4 and/or N2) stage III groups.

## Claims

1. A method for determining, modulating or adjusting a treatment regimen with a chemotherapeutic agent in a patient affected with a colorectal cancer, wherein said agent is able to leverage or promote a tumor-targeted immune response, preferably by causing immunological cell death (ICD),
said method comprising a) quantifying at least two biological markers which are CD8 and CD3 in a tumor sample from the patient, and b) comparing the values obtained in a) to predetermined reference values, and c) deciding to increase the exposure of the patient to the chemotherapeutic agent by deciding to increase the dose, the duration of treatment and/or the frequency of administration, when the values obtained in a) are superior to a predetermined reference value;
wherein step a) is performed by quantifying the density of CD3+ cells in center of the tumor (CT), the density of CD8+ cells in the center of the tumor (CT), the density of CD3+ cells in the invasive margin (IM), and the density of CD8+ cells in the invasive margin (IM);
wherein step b) further comprises classifying the patient in groups (Low, Intermediate, or High Immunoscore (IS)) as follows:
Low IS corresponds to a mean percentile below 25%,
Intermediate IS corresponds to a mean percentile between 25% and 70%,
High IS corresponds to a mean percentile above 70%;
wherein the mean percentile refers to the mean percentile of the 4 individual percentiles of the 4 markers (CD3ct, CD8ct, CD3im, CD8im) corresponding to the density of CD3 cells in CT and IM regions and the density of CD8 cells in CT and IM regions;
wherein the chemotherapeutic agent is oxaliplatin, optionally combined with 5-fluorouracil (5FU) and/or capecitabine;
wherein, when the patient has been classified as belonging to the intermediate or high IS group, deciding to adjust the duration of the treatment regimen with the chemotherapeutic agent to at least six months, and the oxaliplatin dose to at least a cumulative dose of 700 mg/m²

2. The method of claim 1, comprising deciding to adjust the duration of the treatment to six months.

## Patentansprüche

1. Verfahren zur Bestimmung, Modulation oder Anpassung eines Behandlungsschemas mit einem chemotherapeutischen Mittel bei einem Patienten, der an Darmkrebs leidet, wobei dieses Mittel in der Lage ist, eine auf den Tumor gerichtete Immunantwort auszulösen oder zu fördern, vorzugsweise indem es den immunologischen Zelltod (immunological cell death, ICD) verursacht,
dieses Verfahren umfasst a) die Quantifizierung von mindestens zwei biologischen Markern, nämlich CD8 und CD3, in einer Tumorprobe des Patienten und b) den Vergleich der in a) erhaltenen Werte mit vorgegebenen Referenzwerten und c) die Entscheidung, die Exposition des Patienten gegenüber dem chemotherapeutischen Mittel zu erhöhen, durch die Entscheidung, die Dosis, die Behandlungsdauer und/oder die Häufigkeit der Verabreichung zu erhöhen, wenn die in a) erhaltenen Werte über einem vorgegebenen Referenzwert liegen;
wobei Schritt a) durch Quantifizieren der Dichte von CD3+-Zellen im Zentrum des Tumors (CT), der Dichte von CD8+-Zellen im Zentrum des Tumors (CT), der Dichte von CD3+-Zellen im invasiven Rand (IM) und der Dichte von CD8+-Zellen im invasiven Rand (IM) durchgeführt ist;
wobei Schritt b) außerdem die Klassifizierung des Patienten in Gruppen (niedriger, mittlerer oder hoher Immunscore (IS)) wie folgt umfasst:
ein niedriger IS entspricht einem mittleren Durchschnittswert unter 25 %,
ein mittlerer IS entspricht einem mittleren Durchschnittswert zwischen 25 % und 70 %,
ein hoher IS entspricht einem mittleren Durchschnittswert von über 70 %;
wobei sich der mittlere Durchschnittswert auf den mittleren Durchschnittswert der 4 einzelnen Durchschnittswerte der 4 Marker (CD3ct, CD8ct, CD3im, CD8im) bezieht, die der Dichte von CD3-Zellen in CT- und IM-Regionen und der Dichte von CD8-Zellen in CT- und IM-Regionen entsprechen;
wobei das chemotherapeutische Mittel Oxaliplatin ist, optional kombiniert mit 5-Fluorouracil (5FU) und/oder Capecitabin;
wobei, wenn der Patient als zur mittleren oder hohen IS-Gruppe gehörend eingestuft wurde,
die Entscheidung getroffen ist, die Dauer des Behandlungsschemas mit dem chemotherapeutischen Mittel auf mindestens sechs Monate und die Oxaliplatin-Dosis auf mindestens eine kumulative Dosis von 700 mg/m² anzupassen.

2. Verfahren nach Anspruch 1, umfassend die Entscheidung, die Behandlungsdauer auf sechs Monate anzupassen.

## Revendications

1. Méthode pour déterminer, moduler ou ajuster un régime de traitement avec un agent chimiothérapeutique chez un patient affecté d'un cancer colorectal, dans laquelle ledit agent est capable d'optimiser ou de favoriser une réponse immunitaire ciblant une tumeur, de préférence en causant une mort cellulaire immunologique (ICD),
ladite méthode comprenant a) la quantification d'au moins deux marqueurs biologiques qui sont CD8 et CD3 dans un échantillon de tumeur du patient, et b) la comparaison des valeurs obtenues en a) à des valeurs de référence prédéterminées, et c) la décision d'augmenter l'exposition du patient à l'agent chimiothérapeutique par la décision d'augmenter la dose, la durée du traitement et/ou la fréquence d'administration, lorsque les valeurs obtenues en a) sont supérieures à une valeur de référence prédéterminée ;
dans laquelle l'étape a) est effectuée par quantification de la densité des cellules CD3+ au centre de la tumeur (CT), de la densité des cellules CD8+ au centre de la tumeur (CT), de la densité des cellules CD3+ dans la marge invasive (IM), et de la densité des cellules CD8+ dans la marge invasive (IM) ;
dans laquelle l'étape b) comprend en outre le classement du patient dans des groupes (score immunologique (IS) faible, intermédiaire, ou élevé) comme suit :
un IS faible correspond à un percentile moyen inférieur à 25 %,
un IS intermédiaire correspond à un percentile moyen compris entre 25 % et 70 %,
un IS élevé correspond à un percentile moyen supérieur à 70 % ;
dans laquelle le percentile moyen se réfère au percentile moyen des 4 percentiles individuels des 4 marqueurs (CD3ct, CD8ct, CD3im, CD8im) correspondant à la densité des cellules CD3 dans les régions CT et IM et à la densité des cellules CD8 dans les régions CT et IM ;
dans laquelle l'agent chimiothérapeutique est l'oxaliplatine, optionnellement combiné avec du 5-fluorouracile (5FU) et/ou de la capécitabine ;
dans laquelle, lorsque le patient a été classé comme appartenant au groupe IS intermédiaire ou élevé, il est décidé d'ajuster la durée du régime de traitement avec l'agent chimiothérapeutique à au moins six mois, et la dose d'oxaliplatine à au moins une dose cumulative de 700 mg/m².

2. Méthode selon la revendication 1, comprenant la décision d'ajuster la durée du traitement à six mois.
